# FASCICULE DE BREVET EUROPEEN

(11) **EP 2 122 357 B1**
(45) Date de publication et mention de la délivrance du brevet: **21.12.2011**
(21) Numéro de dépôt: 08761852.6
(22) Date de dépôt: 07.02.2008
(51) Int. Cl.: G01N 33/569

(54) **ANTIGENE DIGALACTOLIPIDIQUE EXPOSE A LA SURFACE DES PARASITES APICOMPLEXES ET SES APPLICATIONS DIAGNOSTIQUES ET THERAPEUTIQUES**
AUF DER OBERFLÄCHE VON APIKOMPLEXPARASITEN EXPONIERTES DIGALAKTOLIPIDANTIGEN SOWIE SEINE DIAGNOSTISCHE UND THERAPEUTISCHE VERWENDUNG
DIGALACTOLIPIDIC ANTIGEN EXPOSED ON THE SURFACE OF APICOMPLEX PARASITES, AND DIAGNOSTIC AND THERAPEUTIC USE THEREOF

(30) Priorité: 07.02.2007 FR 0700859
(43) Date de publication de la demande: 25.11.2009
(73) Titulaire: Commissariat à l'Énergie Atomique et aux Énergies Alternatives, 75015 Paris (FR); Centre National de la Recherche Scientifique, 75016 Paris (FR)
(72) Inventeur: BOTTE, Cyrille, F-38100 Grenoble (FR); SAIDANI, Nadia, F-34000 Montpellier (FR); BLOCK, Maryse, F-38640 Claix (FR); DUBREMETZ, Jean-François, F-34090 Montpellier (FR); VIAL, Henri, F-34080 Montpellier (FR); CESBRON-DELAUW, Marie-France, F-38070 La Tronche (FR); MERCIER, Corinne, F-38070 La Tronche (FR); MARECHAL, Eric, F-38000 Grenoble (FR)
(74) Mandataire: Leblois-Préhaud, Hélène Marthe Georgette
(86) Numéro de dépôt international: PCT/FR2008/000149
(87) Numéro de publication internationale: WO 2008/107572

(56) Documents cités:
- US-A1- 2006 147 476
- MARÉCHAL ERIC ET AL: "Synthesis of chloroplast galactolipids in apicomplexan parasites." EUKARYOTIC CELL AUG 2002, vol. 1, no. 4, août 2002 (2002-08), pages 653-656, XP002446002 ISSN: 1535-9778 cité dans la demande
- SONDA ET AL: "Lipid biology of Apicomplexa: perspectives for new drug targets, particularly for Toxoplasma gondii" TRENDS IN PARASITOLOGY, ELSEVIER CURRENT TRENDS, vol. 22, no. 1, janvier 2006 (2006-01), pages 41-47, XP005231189 ISSN: 1471-4922
- MOODY A: "Rapid diagnostic tests for malaria parasites" CLINICAL MICROBIOLOGY REVIEWS, WASHINGTON, DC, US, vol. 15, no. 1, janvier 2002 (2002-01), pages 66-78, XP003007430 ISSN: 0893-8512 cité dans la demande
- SASAI K ET AL: "A chicken anti-conoid monoclonal antibody identifies a common epitope which is present on motile stages of Eimeria, Neospora, and Toxoplasma." THE JOURNAL OF PARASITOLOGY JUN 1998, vol. 84, no. 3, juin 1998 (1998-06), pages 654-656, XP002446274 ISSN: 0022-3395
- MORRISSETTE NAOMI S ET AL: "Characterization of extreme apical antigens from Toxoplasma gondii" EXPERIMENTAL PARASITOLOGY, vol. 79, no. 3, 1994, pages 445-459, XP002446275 ISSN: 0014-4894
- BOTTÉ CYRILLE ET AL: "Subcellular localization and dynamics of a digalactolipid-like epitope in Toxoplasma gondii.", JOURNAL OF LIPID RESEARCH APR 2008, vol. 49, no. 4, April 2008 (2008-04), pages 746-762, ISSN: 0022-2275
- BOUT D T ET AL: "PROSPECTS FOR A HUMAN TOXOPLASMA VACCINE", CURRENT DRUG TARGETS, IMMUNE, ENDOCRINE AND METABOLIC DISORDERS, BENTHAM SCIENCE PUBLISHERS, HILVERSUM, NL, vol. 2, no. 3, 1 October 2002 (2002-10-01) , pages 227-234, XP009033506, ISSN: 1568-0088
- BHOPALE GIRISH M: "Development of a vaccine for toxoplasmosis: current status.", MICROBES AND INFECTION / INSTITUT PASTEUR APR 2003 LNKD- PUBMED:12738002, vol. 5, no. 5, April 2003 (2003-04), pages 457-462, ISSN: 1286-4579
- BISANZ CORDELIA ET AL: "Toxoplasma gondii acyl-lipid metabolism: de novo synthesis from apicoplast-generated fatty acids versus scavenging of host cell precursors.", THE BIOCHEMICAL JOURNAL 15 FEB 2006 LNKD- PUBMED:16246004, vol. 394, no. Pt 1, 15 February 2006 (2006-02-15), pages 197-205, ISSN: 1470-8728

## Description

La présente invention concerne un digalactolipide, exposé à la surface des parasites apicomplexes sous la forme d'un digalactoglycérolipide de type végétal, et apte à induire la production d'anticorps spécifiques, capables d'inhiber la prolifération et/ou les facultés invasives de ces parasites.

La présente invention concerne
- l'utilisation d'un digalactolipide purifié, comprenant le digalactose 6-O-(alpha-D-galactopyrannosyl)-beta-D-galactopyrannose associé de façon covalente à un lipide, comme antigène pour la préparation d'un vaccin destiné à la prévention ou au traitement des infections par les parasites apicomplexes chez l'homme ou l'animal;
- l'utilisation d'un anticorps dirigé contre un tel digalactolipide purifié, ou d'un fragment fonctionnel dudit anticorps comprenant au moins les domaines variables des chaînes lourdes et légères, pour la préparation d'un médicament destiné à la prévention ou au traitement des infections par les parasites apicomplexes chez l'homme ou l'animal;
- une méthode *in vitro* de détection d'une infection par un parasite apicomplexe, comprenant l'immunodétection des parasites apicomplexes ou des anticorps spécifiques desdits parasites présents dans un échantillon biologique issu d'un individu, selon les étapes consistant en:
   (a) la mise en contact dudit échantillon biologique avec au moins un digalactolipide purifié comprenant le digalactose 6-O-(alpha-D-galactopyrannosyl)-beta-D-galactopyrannose associé de façon covalente à un lipide, un anticorps dirigé contre un tel digalactolipide purifié ou un fragment fonctionnel dudit anticorps comprenant au moins les domaines variables des chaînes lourdes et légères d'anticorps, soit directement, soit en même temps que ou après une étape de perméabilisation membranaire dudit échantillon biologique, et
   (b) la révélation des complexes antigène-anticorps formés en (a);
- un digalactolipide purifié, comprenant le digalactose 6-O-(alpha-D-galactopyrannosyl)-beta-D-galactopyrannose associé de façon covalente à un lipide, pour utilisation comme médicament;
- un anticorps dirigé contre un tel digalactolipide purifié, ou d'un fragment fonctionnel dudit anticorps comprenant au moins les domaines variables des chaînes lourdes et légères, pour utilisation comme médicament.

Le phylum des Apicomplexes compte plusieurs milliers de parasites unicellulaires, parmi lesquels des agents de pathologies majeures chez l'homme ou l'animal :
- les agents du paludisme (malaria) chez l'homme (genre *Plasmodium*) *: Plasmodium falciparum, Plasmodium vivax, Plasmodium ovale,* et *Plasmodium malariae,*
- les agents de la piroplasmose (genre *Babesia*)*,* chez l'homme (*Babesia microti*), et l'animal, notamment *B. divergens*, *B. bigemina*, *B. major* et *B. bovis* (bovin), *B. canis* et B. *gibsoni* (chien), *B. equi* et *B. caballi* (cheval),
- l'agent de la toxoplasmose humaine (*Toxoplasma gondii*),
- l'agent de la néosporose chez le chiot et d'autres espèces animales, notamment les bovins (*Neospora caninum*)*,*
- les agents de la cryptosporidiose (genre *Cryptosporidium*)*,* chez l'homme (*Cryptosporidium hominis*) et l'animal (*C. parvum,* par exemple),
- les agents de la theilériose (genre *Theileria*), chez l'animal (bovin, ovin, caprin), notamment *Theileria annulata* et *T. parva*, et
- les agents d'autres maladies infectieuses chez les vertébrés et invertébrés, par exemple : *Sarcocystis neurona* (cheval), *Eimeria tenella* (poulet), et *Gregarina niphandrodes* (invertébrés).

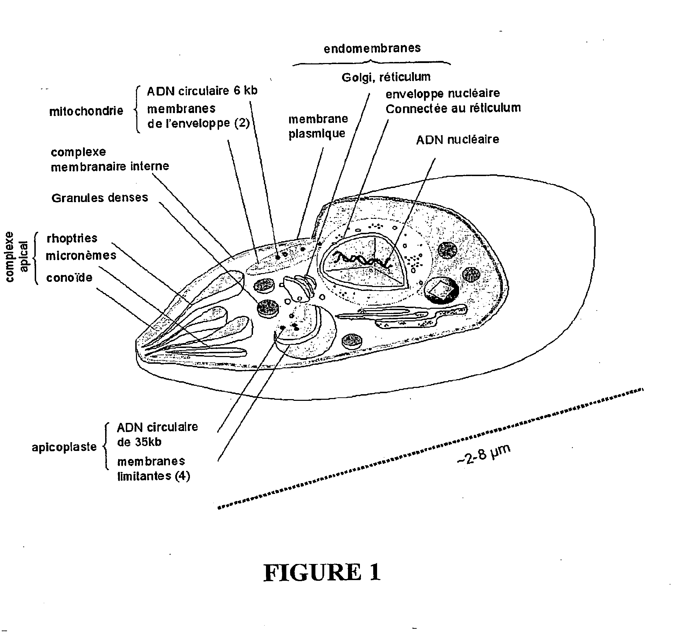

Tous les parasites du phylum des Apicomplexes (parasites apicomplexes ou apicomplexes) partagent un plan d'organisation cellulaire polarisé, avec un pôle basal et un pôle apical. La compartimentation membranaire présente des particularités par rapport à une cellule eucaryote de type levure ou mammifère (Figure 1). D'une part la membrane plasmique est associée à une double membrane, appelée complexe membranaire interne (*Inner Membrane Complex* ou IMC). L'ensemble formé par le complexe membranaire interne et la membrane plasmique constitue la pellicule. D'autre part, dans la partie apicale, la cellule présente des organes sécrétoires uniques (rhoptries, micronèmes et granules denses), sécrétés lors de l'invasion d'une cellule hôte. Enfin, un organite végétal typique, un chloroplaste résiduel non photosynthétique appelé apicoplaste, se retrouve dans la cellule (McFadden et al., Nature, 1996, 381, 482; Kôhler et al., Science, 1997, 275, 1485-1489 ; revue dans Maréchal E. et M.F. Cesbron-Delauw, Trends Plant Sci., 2001, 6, 200-205).

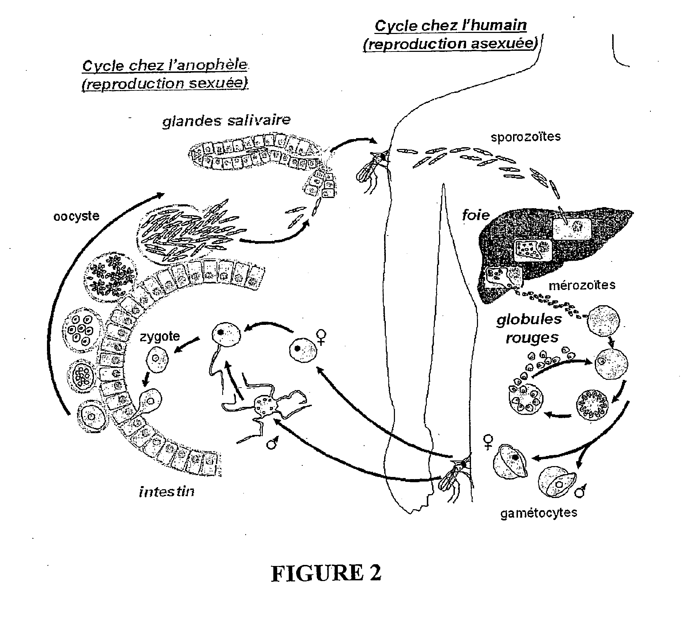

Ces parasites sont transmis par différents vecteurs. Par exemple, le vecteur du paludisme est le moustique femelle Anophèle (Figure 2). Le *Plasmodium* pénètre dans le corps par le sang, dans lequel il évolue sous forme libre appelée sporozoïte. Plus tard dans le cycle du parasite, *Plasmodium* passe par une forme intracellulaire dans les hépatocytes, puis par une forme de multiplication dans les globules rouges. Lors de son passage d'un globule rouge à un autre, transition qui se produit environ tous les 48 heures, au moment des crises de fièvre paludiques, le *Plasmodium* évolue sous une autre forme sanguine libre appelée mérozoïte.

La mise au point d'un vaccin sous-unitaire et d'un test d'immunodétection efficaces contre les infections par les parasites apicomplexes, et en particulier les infections par tous les *Plasmodium* responsables du paludisme, s'est révélée extrêmement difficile du fait de la présence de nombreux antigènes, de la spécificité de ces antigènes pour une espèce particulière de parasite et un stade particulier du cycle des parasites, ainsi que de l'existence de mécanismes d'échappement des parasites à la réponse immunitaire de l'hôte humain.

Par exemple, les principaux tests d'immunodétection rapide du paludisme reposent sur la détection d'antigènes protéiques de surface (HRP-2) ou sécrétés (antigène soluble ; pLDH (plasmodium Lactate Dehydrogenase) et aldolase), par immunochromatographie (revue dans A. Moody, Clinical Microbiology Reviews, 2002,15, 66-78).

HRP-2 est un antigène exprimé à la surface des érythrocytes et spécifique des stades asexués et des jeunes gamétocytes de *P. falciparum* ; les tests d'immunodétection d'HRP-2 permettent de détecter uniquement les infections par P. *falciparum,* mais pas les infections par *Plasmodium vivax, Plasmodium ovale,* et *Plasmodium malariae.*

L'aldolase et la pLDH sont des enzymes glycolytiques solubles, exprimées par les quatre espèces de *Plasmodium* responsables de la malaria. Toutefois la sensibilité des tests d'immunodétection est faible vis-à-vis de *Plasmodium ovale,* et *Plasmodium malariae,* dans la mesure où les anticorps monoclonaux utilisés présentent une faible affinité pour les antigènes de ces deux espèces de *Plasmodium.*

Un épitope invariant de la surface des cellules parasitaires et un anticorps dirigé contre cet épitope seraient intéressants tant du point de vue diagnostique que prophylactique et thérapeutique.

Contrairement aux épitopes peptidiques qui sont soumis à une variation génétique rapide, les épitopes non-peptidiques portés par des glycoprotéines ou des lipides (glycolipides, glycophospholipides) de surface des parasites sont plus stables et mieux conservés.

Toutefois, parmi ces molécules de surface, seules quelques unes sont immunogènes chez l'animal. Par exemple, dans le cas des phospholipides classiques tels que la phosphatidylcholine et la phosphatidyléthanolamine il n'est pas possible d'éveiller le système immunitaire contre ces composés que l'on trouve chez tous les eucaryotes, et en particulier les mammifères.

Ainsi, les principaux épitopes non-peptidiques de surface des parasites apicomplexes qui ont été identifiés et caractérisés sont portés par des glycophospholipides, et en particulier les glycophosphatidylinositols (GPIs) de surface de *Plasmodium*. Il a notamment été montré que des anticorps dirigés contre une région conservée des groupements carbohydrate des GPIs de *P. falciparum* neutralisent les effets toxiques de ces GPIs. Ainsi, l'immunisation avec un GPI ou un carbohydrate synthétique dérivé d'un GPI de *Plasmodium* représente une stratégie vaccinale pour prévenir les formes sévères de la malaria (Schofield et al., Nature, 2002, 418, 785-789; Naik et al., Infect. Immun., 2006, 74, 1412-1415). Toutefois, cette stratégie vaccinale ne permet pas de prévenir l'infection par les *Plasmodium.*

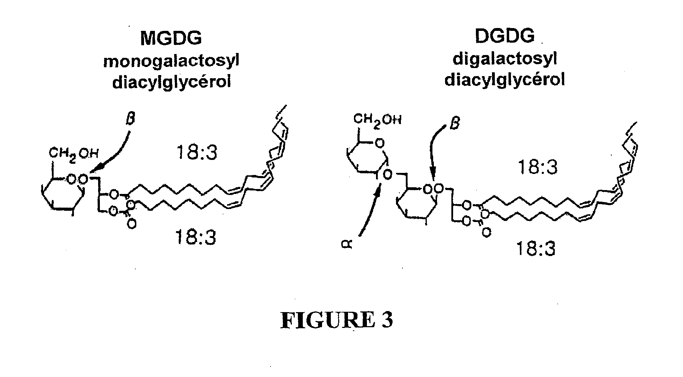

Les galactoglycérolipides et notamment les plus abondants tels que le monogalactosyldiacylglycérol (MGDG) et le digalactosyldiacylglycérol (DGDG) sont des lipides spécifiques des plastes végétaux, les organites cytoplasmiques des cellules végétales effectuant la photosynthèse. La structure du MGDG et du DGDG comprend une tête polaire avec un (MGDG) ou deux (DGDG) galactoses, en position 3 du glycérol (Figure 3). A ce jour, ces deux galactoglycérolipides n'ont été détectés que chez les cyanobactéries (ancêtre des plastes) et dans les cellules d'eucaryotes porteurs de plastes (plantes, algues et protistes tels que les apicomplexes).

Chez les parasites apicomplexes, les galactoglycérolipides sont peu abondants et indétectables par les méthodes classiques d'extraction des lipides et de séparation par chromatographie en couche mince à deux dimensions (Maréchal et al. Eukaryot. Cell., 2002, 1, 653-656). Toutefois, le marquage radioactif des galactoglycérolipides en présence DUDP-[³H]galactose (Maréchal *et al.*, 2002, précité) ou de [¹⁴C]acétate (Bisanz et al., Biochem. J., 2006, 394, 177-205) a permis de mettre en évidence la synthèse de deux types de galactoglycérolipides par les parasites apicomplexes : un monogalactolipide de structure compatible avec un diradylglycérol, soit du MGDG, soit du MGAAG (monogalactosylalkylacylglycérol) et un digalactolipide de structure compatible avec un diradylglycérol, soit du DGDG, soit du DGAAG (digalactosylalkylacylglycérol).

Les Inventeurs ont montré que le digalactolipide des parasites apicomplexes et plus particulièrement le digalactose porté par ce digalactolipide est un antigène utile pour le diagnostic et la vaccination des infections par l'ensemble des parasites apicomplexes.

En effet, les anticorps anti-digalactolipide, notamment les anticorps anti-DGDG, reconnaissent spécifiquement l'ensemble des stades parasitaires des apicomplexes et sont donc utiles pour le diagnostic de l'ensemble des infections par ces parasites, chez l'homme ou l'animal.

Des expériences d'immunomarquage des parasites apicomplexes (parasites libres), sans perméabilisation des membranes cellulaires des parasites, ont montré que la localisation du digalactolipide réagissant avec l'anticorps anti-digalactolipide, notamment un anticorps anti-DGDG, n'était pas uniquement intracellulaire (plaste) mais également à la surface externe de la membrane plasmique des parasites apicomplexes.

Cet antigène digalactolipidique qui est présent à la surface externe du parasite peut donc être détecté directement à la surface des parasites libres, sans étape de perméabilisation membranaire.

De plus, le digalactolipide, notamment le DGDG, est capable d'induire la production d'anticorps protecteurs, aptes à inhiber la prolifération et les facultés invasives de ces parasites. De tels anticorps sont capables d'agglutiner les parasites vivants et empêchent (neutralisation) l'invasion des cellules hôtes par ces parasites. Ils facilitent également la phagocytose des parasites et activent la voie classique du complément. Par conséquent, le digalactolipide, notamment le DGDG est un antigène utile pour la vaccination contre les infections par l'ensemble des parasites apicomplexes. De même les anticorps dirigés contre le digalactolipide, notamment le DGDG, sont utiles en immunothérapie passive (sérothérapie), contre l'ensemble de ces infections.

En outre, des expériences complémentaires ont montré que la fraction antigénique du digalactoglycérolipide des parasites apicomplexes était constituée par la partie digalactose associée de façon covalente au lipide. En effet, l'absence de réactivité des sérums anti-DGDG avec le MGDG et le tri-DGD indique que les anticorps dirigés contre le DGDG reconnaissent spécifiquement le digalactoside. En outre, l'immunisation avec le MGDG, dans des conditions semblables à celles du DGDG, indique que le MGDG n'est pas capable d'induire une réponse anticorps spécifique.

En conséquence, la présente invention a pour objet
- l'utilisation d'un digalactolipide purifié, comprenant le digalactose 6-O-(alpha-D-galactopyrannosyl)-beta-D-galactopyrannose associé de façon covalente à un lipide, comme antigène pour la préparation d'un vaccin destiné à la prévention ou au traitement des infections par les parasites apicomplexes chez l'homme ou l'animal;
- l'utilisation d'un anticorps dirigé contre un tel digalactolipide purifié, ou d'un fragment fonctionnel dudit anticorps comprenant au moins les domaines variables des chaînes lourdes et légères, pour la préparation d'un médicament destiné à la prévention ou au traitement des infections par les parasites apicomplexes chez l'homme ou l'animal;
- une méthode *in vitro* de détection d'une infection par un parasite apicomplexe, comprenant l'immunodétection des parasites apicomplexes ou des anticorps spécifiques desdits parasites présents dans un échantillon biologique issu d'un individu, selon les étapes consistant en:
   (a) la mise en contact dudit échantillon biologique avec au moins un digalactolipide purifié comprenant le digalactose 6-O-(alpha-D-galactopyrannosyl)-beta-D-galactopyrannose associé de façon covalente à un lipide, un anticorps dirigé contre un tel digalactolipide purifié ou un fragment fonctionnel dudit anticorps comprenant au moins les domaines variables des chaînes lourdes et légères d'anticorps, soit directement, soit en même temps que ou après une étape de perméabilisation membranaire dudit échantillon biologique, et
   (b) la révélation des complexes antigène-anticorps formés en (a);
- un digalactolipide purifié, comprenant le digalactose 6-O-(alpha-D-galactopyrannosyl)-beta-D-galactopyrannose associé de façon covalente à un lipide, pour utilisation comme médicament;
- un anticorps dirigé contre un tel digalactolipide purifié, ou d'un fragment fonctionnel dudit anticorps comprenant au moins les domaines variables des chaînes lourdes et légères, pour utilisation comme médicament.

Au sens de la présente invention on entend par antigène digalactolipidique un antigène comprenant le digalactose 6-O-(alpha-D-galactopyrannosyl)-beta-D-galactopyrannose associé de façon covalente à un lipide ou une molécule appropriée dérivée d'un lipide, ledit antigène étant capable d'induire la production d'anticorps : (i) dirigés contre ce digalactose, (ii) reconnaissant spécifiquement un digalactolipide de parasite(s) apicomplexe(s), et (iii) capables d'inhiber la prolifération et/ou les facultés invasives de ce(s) parasite(s) apicomplexe(s) qui sont reconnus par ledit anticorps.

L'inhibition de la prolifération et/ou des facultés invasives des parasites apicomplexes peut-être mesurée *in vitro* par un test classique de croissance de parasite(s) apicomplexe(s) en cellules humaines ou animales, notamment par un test colorimétrique ou test de fluorescence, selon le principe décrit pour *Toxoplasma gondii* (Mc Fadden et al., Antimicrob. Agents Chemother., 1997, 41, 1849-1853 ; Seebert et al., Gene, 1996, 169, 39-45 ; Gubbels et al., Antimicrob. Agents Chemother., 2003, 47, 309-316).

Alternativement, l'inhibition de la prolifération et/ou des facultés invasives des parasites apicomplexes peut-être mesurée *in vitro* par un test classique d'opsonisation (phagocytose par les macrophages), de façon à mettre en évidence des anticorps qui augmentent la phagocytose des parasites apicomplexes par les macrophages (anticorps opsonisants).

L'antigène selon l'invention comprend le digalactose 6-O-(alpha-D-galactopyrannosyl)-beta-D-galactopyrannose associé de façon covalente à un lipide ou à une molécule appropriée dérivée d'un lipide, de façon à induire la production d'anticorps spécifiques. Le digalactose peut notamment être couplé à une molécule hydrophobe connue de l'homme du métier comme un lipide, ou bien inclus dans cette molécule hydrophobe (glycolipide). Le digalactose peut également être conjugué à tout support connu de l'homme du métier comme présentant des propriétés d'hydrophobicité propres aux lipides, selon les techniques conventionnelles de synthèse et de couplage moléculaires.

Au sens de la présente invention, on entend par anticorps dirigé contre un antigène digalactosidique ou anticorps anti-digalactose, un anticorps produit par immunisation d'un vertébré, en particulier un mammifère, avec un antigène digalactolipique.

Au sens de la présente invention on entend par fragment d'anticorps un fragment fonctionnel dudit anticorps comprenant au moins les domaines variables des chaînes lourdes et légères, tels que notamment les fragments Fab, Fv et scFv.

Au sens de la présente invention, on entend par anticorps chimérique, relativement à un anticorps d'une espèce animale particulière ou d'une classe particulière d'anticorps, un anticorps comprenant tout ou partie d'une chaîne lourde et/ou d'une chaîne légère d'un anticorps d'une autre espèce animale ou d'une autre classe d'anticorps.

Au sens de la présente invention, on entend par anticorps humanisé une immmunoglobuline humaine dans laquelle les résidus des CDRs (*Complementary-Determining Regions*) qui forment le site de liaison à l'antigène sont remplacés par ceux d'un anticorps monoclonal non-humain possédant la spécificité, l'affinité ou l'activité recherchées.

Le digalactolipide selon l'invention, qui est apte à induire la production d'anticorps spécifiques, capables d'inhiber la prolifération et/ou les facultés invasives des parasites apicomplexes est utilisé comme antigène pour la vaccination contre les infections par ces parasites.

En outre ce digalactolipide qui est apte à être reconnu par des anticorps spécifiques des parasites apicomplexes est utilisé pour le diagnostic sérologique (diagnostic indirect) des infections par les parasites du phylum des Apicomplexes chez l'homme ou l'animal, notamment pour des études épidémiologiques ; les anticorps spécifiques éventuellement présents dans les sécrétions, notamment le sérum des individus, sont détectés par une technique d'immunochimie appropriée, notamment EIA, ELISA, RIA, immunofluorescence, immunoagglutination, immunochromatographie, immunocytochimie, immunohistochimie, immunoblot, ou immunoprécipitation, à l'aide d'antigène digalactolipidique purifié selon les techniques classiques connues de l'Homme du métier.

Les anticorps et les fragments d'anticorps tels que définis ci-dessus, de préférence purifiés selon les techniques classiques connues de l'Homme du métier, sont utilisés pour traiter et détecter les infections par les parasites du phylum des Apicomplexes (diagnostic direct) chez l'homme ou l'animal.

La détection des parasites est réalisée par une technique d'immunochimie appropriée, notamment EIA, ELISA, RIA, immunofluorescence, immunoagglutination, immunochromatographie, immunocytochimie, immunohistochimie, immunoblot, ou immunoprécipitation, à partir d'un échantillon biologique (sang, sérum, fèces, urine, liquide céphalo-rachidien), prélevé chez un individu susceptible d'être infecté.

Les anticorps dirigés contre le digalactolipide selon l'invention, qui sont capables d'inhiber la prolifération et/ou les facultés invasives des parasites apicomplexes sont utilisés en sérothérapie (immunothérapie passive), pour traiter les infections par ces parasites. La sérothérapie humaine est de préférence réalisée avec des anticorps humanisés, tels que définis ci-dessus.

Par comparaison avec les anticorps non-humains, les anticorps humanisés sont moins immunogènes et possèdent une demi-vie prolongée chez l'Homme car ils ne possèdent qu'une faible proportion de séquences non-humaines étant donné que la quasi-totalité des résidus des régions FR (*Framework*) et de la région constante (Fc) de ces anticorps sont ceux d'une séquence consensus d'immunoglobulines humaines.

L'invention englobe l'utilisation d'anticorps monoclonaux ou polyclonaux, d'anticorps chimériques tels que les anticorps humanisés, ainsi que de leurs fragments (Fab, Fv, scFv).

L'invention englobe le diagnostic et la vaccination spécifiques d'infections par des parasites apicomplexes (Apicomplexa), responsables d'une pathologie chez l'Homme ou l'animal (vertébré ou invertébré), tels que notamment :
- les agents du paludisme (malaria) chez l'homme (genre *Plasmodium*) *: Plasmodium falciparum, Plasmodium vivax, Plasmodium ovale,* et *Plasmodium malariae,*
- les agents de la piroplasmose (genre *Babesia*)*,* chez l'homme (*Babesia microti*)*,* et l'animal, par exemple: *B. divergens, B. bigemina, B. major* et *B. bovis* (bovin), *B. canis* et B. *gibsoni* (chien), *B. equi,* et *B. caballi* (cheval),
- l'agent de la toxoplasmose humaine (*Toxoplasma gondii*)*,*
- l'agent de la néosporose chez le chiot et d'autres espèces animales, notamment les bovins (*Neospora caninum*)*,*
- les agents de la cryptosporidiose (genre *Cryptosporidium),* chez l'homme (*Cryptosporidium hominis*) et l'animal (*C. parvum,*)*,*
- les agents de la theilériose (genre *Theileria*)*,* chez l'animal (bovin, ovin, caprin), par exemple : *Theileria annulata* et *T. parva,* et
- les agents d'autres maladies infectieuses chez les vertébrés et invertébrés, par exemple : *Sarcocystis neurona* (*cheval*)*, Eimeria tenella* (poulet), et *Gregarina niphandrodes* (invertébrés).

Selon un mode de réalisation avantageux de ladite utilisation, ledit antigène digalactolipidique comprend le digalactose 6-O-(α-D-galactopyrannosyl)-β-D-galactopyrannose, associé de façon covalente à un lipide.

Selon une disposition avantageuse de ce mode de réalisation, le digalactosyldiradylglycéride de formule I est le 1,2-diacyl-(α-D-galactopyranosyl-(1→6)-β-D-galactopyranosyl-(1'→ 3)-*sn*-glycerol (DGDG).

Un réactif de diagnostic d'une infection par un parasite apicomplexe, caractérisé en ce qu'il est constitué par un antigène digalactolipidique tel que défini ci-dessus, un anticorps dirigé contre ledit antigène ou un fragment dudit anticorps, tels que définis ci-dessus est également décrit.

Les réactifs peuvent être couplés à des particules ou à un marqueur approprié. Les particules sont notamment des particules d'or, des liposomes (liposomes contenant un colorant) ou des érythrocytes (immunoagglutination). Le marqueur est une substance qui produit un signal détectable par les méthodes conventionnelles ou qui réagit avec une autre substance, de façon à produire un signal détectable. Parmi les marqueurs utilisables dans les méthodes de diagnostic, on peut citer notamment, les chromophores, les substances fluorescentes ou chimioluminescentes, les enzymes, les isotopes radioactifs et les agents complexants (Protéine A, Protéine G, biotine, lectine).

Les réactifs tels que définis ci-dessus peuvent également être immobilisés sur un support approprié, selon les méthodes classiques connues de l'homme du métier. Parmi les supports appropriés sur lesquels peuvent être immobilisés ces réactifs on peut citer notamment ceux en matière plastique (latex, polystyrène, polyvinylchloride, polyuréthane, polyacrylamide, polyvinylacétate), en verre, ou en papier (nitrocellulose). Ces supports sont sous la forme de plaques (microplaques), de feuilles, de bandelette, ou de particules (billes). L'immobilisation des réactifs sur le support peut être réalisée par formation d'un pontage entre le réactif et une molécule qui est couplée au support (hydrazide, Protéine A, glutaraldéhyde, carboiimide ou lysine), selon les techniques conventionnelles.

La présente invention a également pour objet une méthode *in vitro* de détection d'une infection par un parasite apicomplexe, comprenant l'immunodétection des parasites apicomplexes ou des anticorps spécifiques desdits parasites présents dans un échantillon biologique issu d'un individu, selon les étapes consistant en:
(a) la mise en contact dudit échantillon biologique avec au moins un digalactolipide purifié comprenant le digalactose 6-O-(alpha-D-galactopyrannosyl)-beta-D-galactopyrannose associé de façon covalente à un lipide, un anticorps dirigé contre un tel digalactolipide purifié ou un fragment fonctionnel dudit anticorps comprenant au moins les domaines variables des chaînes lourdes et légères d'anticorps, soit directement, soit en même temps que ou après une étape de perméabilisation membranaire dudit échantillon biologique, et
(b) la révélation des complexes antigène-anticorps formés en (a).

L'invention englobe les méthodes d'immunochimie conventionnelles, par exemple : EIA, ELISA, RIA, immunofluorescence, immunoagglutination, immunochromatographie, immunocytochimie, immunohistochimie, immunoblot, et immunoprécipitation.

Selon un mode de réalisation avantageux de ladite méthode, l'étape (a) comprend la mise en contact dudit échantillon biologique, successivement avec au moins un premier anticorps de capture dirigé contre l'antigène digalactolipidique et au moins un second anticorps spécifique du parasite à détecter et différent du premier anticorps, ou bien avec un fragment du premier et/ou du second anticorps. L'utilisation d'un deuxième anticorps spécifique de genre (*Plasmodium,* par exemple) ou d'espèce (*P. falciparum* ou *P. vivax*, par exemple) permet avantageusement de déterminer le genre ou l'espèce de parasite responsable de l'infection.

Selon un autre mode de réalisation avantageux de ladite méthode, elle comprend préalablement ou de façon concomitante à l'étape a), une étape de perméabilisation membranaire.

La perméabilisation membranaire des cellules hôtes permet de détecter les stades intracellulaires du parasite. La perméabilisation membranaire des parasites permet de détecter les antigènes internes (non-exposés à la surface de la membrane plasmique du parasite).

La perméabilisation membranaire est effectuée dans les conditions standard, selon les méthodes classiques connues de l'Homme du métier. Elle peut notamment être effectuée en présence de détergent, comme le Triton-X100, à des concentrations de l'ordre de 0,1 %.

Ladite méthode est avantageusement une méthode ELISA d'immunocapture dans laquelle :
- l'étape (a) comprend :
   (a₁) la mise en contact dudit échantillon biologique avec au moins un premier anticorps ou un fragment d'anticorps dirigé contre l'antigène digalactolipidique tel que défini ci-dessus, qui est fixé sur un support approprié, notamment une microplaque,
   (a₂) le lavage de la phase solide, et
   (a₃) l'addition d'au moins un second anticorps ou un fragment d'anticorps, différent du premier, ledit anticorps ou fragment d'anticorps étant éventuellement marqué de façon appropriée, et
- l'étape (b) de révélation des complexes antigène-anticorps formés est réalisée, soit directement à l'aide d'un second anticorps marqué par exemple avec de la biotine ou une enzyme appropriée telle que la peroxydase ou la phosphatase alcaline, soit indirectement à l'aide d'un sérum anti-immunoglobulines marqué comme ci-dessus. Les complexes ainsi formés sont révélés à l'aide d'un substrat approprié.

Par exemple :
- l'étape (a₁) est réalisée avec au moins un premier anticorps monoclonal ou polyclonal ou un fragment de ceux-ci, dirigé contre l'antigène digalactolipidique, tel que défini ci-dessus et
- l'étape (a₃) est réalisée avec au moins un anticorps ou un fragment d'anticorps dirigé contre un autre antigène spécifique du parasite du phylum des Apicomplexes à détecter ; lorsqu'un anticorps ou un fragment d'anticorps dirigé contre un antigène interne est utilisé, le dit anticorps est incubé en présence de détergent, comme le Triton X-100 par exemple, à des concentrations de l'ordre de 0,1 %.

Alternativement, ladite méthode est une méthode d'immunochromatographie. Dans une première étape, les parasites éventuellement présents dans l'échantillon biologique sont tout d'abord capturés en phase liquide par un anticorps ou un fragment d'anticorps de capture (par exemple un anticorps conjugué à des particules d'or ou à des liposomes contenant un colorant à base de sélénium), dirigé contre l'antigène digalactolipidique tel que défini ci-dessus. Dans une seconde étape, les complexes antigène-anticorps formés migrent le long de la bandelette de nitrocellulose et sont ensuite capturés par un second anticorps et éventuellement un troisième anticorps (dans le sens de la migration), spécifiques du parasite à détecter et différents du premier anticorps, qui sont immobilisés sur une bandelette de nitrocellulose, d'où l'apparition d'une ligne colorée au niveau du second et éventuellement du troisième anticorps.

L'utilisation de deux anticorps différents immobilisés sur la bandelette de nitrocellulose, le premier ayant une spécificité supérieure ou égale au second, permet de typer le genre (*Plasmodium,* par exemple) et/ou l'espèce (*P. falciparum* ou *P. vivax,* par exemple) de parasite qui est responsable de l'infection. Par exemple, le premier anticorps et le second anticorps sont respectivement : un anticorps spécifique de genre et un anticorps pan-spécifique ; un anticorps spécifique d'espèce (*P*. *falciparum*, par exemple) et un anticorps spécifique de genre (*Plasmodium*, par exemple) ; deux anticorps dirigés contre des espèces différentes (*P. falciparum* et *P. vivax*, par exemple). En outre un anticorps contrôle, immobilisé en aval des anticorps précédents, permet de vérifier que les anticorps présents dans la phase liquide ont bien migré le long de la bandelette.

Un kit de détection d'une infection par un parasite du phylum des Apicomplexes, est également décrit caractérisé en ce qu'il comprend au moins un antigène digalactolipidique ou au moins un anticorps ou un fragment d'anticorps, tels que définis ci-dessus. Le kit comprend avantageusement au moins un second anticorps tel que défini ci-dessus, de préférence un second anticorps spécifique du genre ou de l'espèce du parasite à détecter.

La présente invention a également pour objet un anticorps dirigé contre un antigène digalactolipidique ou un fragment dudit anticorps, tels que définis ci-dessus, pour utilisation comme médicament dans la prévention ou le traitement d'une infection par un parasite du phylum des Apicomplexes.

Une composition pharmaceutique, caractérisée en ce qu'elle comprend au moins un anticorps dirigé contre un antigène digalactolipidique ou un fragment dudit anticorps, tels que définis ci-dessus, un véhicule pharmaceutiquement acceptable et éventuellement une substance porteuse est décrite.

Est aussi décrite, une composition immunogène ou vaccinale, caractérisée en ce qu'elle comprend un antigène digalactolipidique tel que défini ci-dessus, éventuellement associé à un ou plusieurs antigènes d'intérêt, notamment des antigènes spécifiques de parasites apicomplexes, un véhicule pharmaceutiquement acceptable et éventuellement un adjuvant ou une substance porteuse.

Les compositions se présentent sous une forme galénique adaptée à une administration par voie parentérale (sous-cutanée, intramusculaire, intradermique, intraveineuse), entérale (orale, sublinguale), ou locale (nasale, rectale, vaginale).

Les compositions comprennent une dose efficace d'antigène vaccinant ou d'anticorps. La dose efficace d'antigène est la dose nécessaire pour induire une réponse en anticorps spécifiques, capable de prévenir, d'atténuer ou de traiter l'infection parasitaire et les symptômes associés. La dose efficace d'anticorps est la dose capable de prévenir, d'atténuer ou de traiter l'infection parasitaire et les symptômes associés. La dose efficace dépend du type d'agent pathogène, de la voie d'administration et du rythme des administrations, du type de mammifère à traiter, ainsi que d'autres facteurs, connus de l'Homme du métier.

Les véhicules pharmaceutiquement acceptables, les substances porteuses, et les adjuvants, sont ceux classiquement utilisés.

Les adjuvants sont avantageusement choisis dans le groupe constitué par : l'hydroxyde d'alumine, le squalène, la saponine, des émulsions huileuses, des substances minérales, des extraits bactériens (BCG, PPD, toxoide), des cytokines..

Les substances porteuses sont avantageusement sélectionnées dans le groupe constitué par : les liposomes unilamellaires ou multilamellaires, les ISCOMS, les virosomes, les pseudoparticules virales, les micelles de saponine, les microsphères solides de nature saccharidique (poly(lactide-co-glycolide)) ou aurifère, et les nano-particules.

Un anticorps monoclonal ou chimérique ou un fragment dudit anticorps dirigé contre un antigène digalactosidique tels que définis ci-dessus sont également décrits. De préférence, il s'agit d'un anticorps humanisé ou d'un fragment dudit anticorps.

L'antigène digalactolipidique et les anticorps présentent les avantages suivants par rapport aux antigènes/anticorps de l'art antérieur :
- l'antigène est présent à tous les stades parasitaires, chez tous les parasites apicomplexes contenant des plastes. Il est détectable sur les parasites libres, sans étape de perméabilisation membranaire. La détection de l'antigène ou des anticorps dirigés contre cet antigène permet donc le diagnostic de l'ensemble des infections par les parasites apicomplexes, chez l'homme et l'animal,
- l'antigène induit une immunité contre l'infection par l'ensemble des parasites apicomplexes. Un seul vaccin comprenant cet antigène permet de réduire l'incidence des infections par l'ensemble des parasites apicomplexes et les pathologies associées, chez l'homme et chez l'animal.

En outre, parmi ces antigènes, le DGDG présente l'avantage supplémentaire d'être disponible en grande quantité chez les plantes et sa purification est simple, ce qui permet de limiter les coûts de production des réactifs de diagnostic et des vaccins.

Ces différents réactifs sont préparés et utilisés selon les techniques classiques de biochimie et d'immunologie, en suivant les protocoles standards tels que ceux décrits dans Current Protocols in Immunology (John E. Cologan, 2000, Wiley and Son Inc. Library of Congress, USA) et dans Antibodies : A Laboratory Manual (E. Howell and D Lane, Cold Spring Harbor Laboratory, 1988)

De manière plus précise :
- le DGDG est extrait à partir de plantes puis purifié selon les méthodes classiques telles que décrites notamment dans Andersson et al., Journal of Chromatography A, 1997, 785, 337-343 ; Deschamps et al., Journal of Chromatography A, 2004, 1040, 115-121 ; Aro et al., Journal of Cereal Science, 2007, 45, 116-119.
- les anticorps polyclonaux sont préparés par immunisation d'un animal approprié avec un antigène tel que défini ci-dessus, éventuellement conjugué à une protéine porteuse telle que la KLH ou à l'albumine et/ou associé à un adjuvant approprié tel que l'adjuvant de Freund (complet ou incomplet) ou l'hydroxyde d'alumine ; après obtention d'un titre en anticorps satisfaisant, les anticorps sont récoltés par prélèvement du sérum des animaux immunisés et enrichis en immunoglobulines par précipitation, selon les techniques classiques, puis les immunoglobulines spécifiques sont éventuellement purifiées par des techniques appropriées connues de l'Homme du métier, notamment par chromatographie sur une colonne sur laquelle est fixé de la protéine A ou l'antigène tel que défini ci-dessus, de façon à obtenir une préparation d'immunoglobulines monospécifiques.
- les anticorps monoclonaux sont produits à partir d'hybridomes obtenus par fusion de lymphocytes B d'un animal immunisé par l'antigène tel que défini ci-dessus avec des myélomes, selon la technique de Köhler et Milstein (Nature, 1975, 256, 495-497) ; les hybridomes sont cultivés *in vitro*, notamment dans des fermenteurs ou produits *in vivo*, sous forme d'ascite ; alternativement, lesdits anticorps monoclonaux sont produits par génie génétique comme décrit dans le brevet américain US 4,816,567. Par exemple, des animaux sont immunisés de façon forte et répétée avec l'antigène tel que défini ci-dessus, selon un protocole standard comprenant une première immunisation par injection sous-cutanée multisite de l'antigène dans un volume équivalent d'adjuvant complet de Freund puis 10 jours plus tard, une série de quatre immunisations de rappel à 2 semaines d'intervalle, cette fois avec de l'adjuvant incomplet de Freund ; les deux premières immunisations sont effectuées par injection sous-cutanée et intramusculaire et les deux dernières par injection intramusculaire et intraveineuse. Les anticorps monoclonaux sont produits selon un protocole standard comprenant le sacrifice des animaux deux semaines après le dernier rappel, le prélèvement de la rate, la mise en suspension des lymphocytes spléniques et la fusion de ces lymphocytes avec la lignée cellulaire SP2/0 (cette lignée murine ne produit aucun anticorps murin, est immortalisée, et possède toute la machinerie de sécrétion nécessaire à la sécrétion d'immunoglobulines).

- les fragments d'anticorps sont produits à partir des régions V_{H} et V_{L} clonées, à partir des ARNm d'hybridomes ou de lymphocytes spléniques d'un animal immunisé avec l'antigène tel que défini ci-dessus; par exemple, les fragments Fv, scFv ou Fab sont exprimés à la surface de phages filamenteux selon la technique de Winter et Milstein (Nature, 1991, 349, 293-299) ; après plusieurs étapes de sélection, les fragments d'anticorps spécifiques de l'antigène sont isolés et les ADNc correspondant auxdits fragments exprimés dans un système d'expression approprié, par les techniques classiques de clonage et d'expression d'ADN recombinant.
- les anticorps humanisés sont produits par des méthodes générales comme celles décrites dans la Demande Internationale WO 98/45332.

Les anticorps monoclonaux et polyclonaux ou leurs fragments tels que définis ci-dessus, sont purifiés par les techniques classiques connues de l'Homme du métier, notamment par chromatographie sur une colonne sur laquelle est fixé de la protéine A ou l'antigène digalactolipidique tel que défini ci-dessus.

Outre les dispositions qui précèdent, l'invention comprend encore d'autres dispositions, qui ressortiront de la description qui va suivre, qui se réfère à des exemples de mise en oeuvre de l'objet de la présente invention, avec référence aux dessins annexés dans lesquels :
- la figure 1 est un schéma de l'organisation d'une cellule apicomplexe.
- la figure 2 illustre le cycle de *Plasmodium falciparum,* l'agent du paludisme.
- la figure 3 illustre la structure du monogalactosyldiacylglycérol (MGDG) et du digalactosyldiacylglycérol (DGDG), deux galactolipides majeurs des plastes de plantes et d'algues, et des membranes de cyanobactéries. Les acides gras estérifiés aux positions 1 et 2 du glycérol sont des exemples: les longueurs de chaînes carbonées, et le nombre et position de doubles liaisons, ainsi que la présence éventuelle d'oxydation, peuvent varier.
- la figure 4 illustre la spécificité d'un sérum obtenu par immunisation d'un lapin avec du DGDG purifié à partir de chloroplastes d'épinard.

**A.** Le sérum dilué au 100^{ème} a été testé sur une série de lipides purs et de lipides totaux d'enveloppe de chloroplastes d'épinard, ainsi que de lipides totaux extraits de *Toxoplasma gondii* et de *Plasmodium falciparum,* déposés sur une membrane de nitrocellulose. MGDG: monogalactosyldiacylglycérol. DGDG: digalactosyldiacylglycérol. TriGDG : trigalactosyldiacylglycérol. DAG : diacylglycérol. PG : phosphatidylglycérol. PC : phosphatidylcholine. SL : sulfolipide. PE : phosphatidyléthanolamine. SM : sphingomyéline.

**B.** La réactivité de l'anticorps est abolie après préincubation en présence de DGDG purifié à partir de chloroplastes d'épinard.

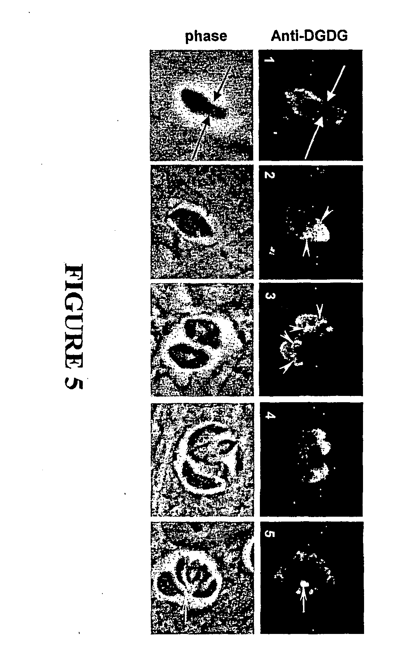

- la figure 5 illustre l'immunomarquage de *Toxoplasma gondii* à l'intérieur de fibroblastes humains, à l'aide d'un sérum anti-DGDG.

Les panneaux supérieurs (1 à 5) illustrent l'analyse du marquage par le sérum anti-DGDG, en microscopie de fluorescence. Les panneaux inférieurs représentent la visualisation des cellules en microscopie de phase. **1.** Pénétration d'un *Toxoplasma* libre dans une cellule humaine (au niveau du point de constriction indiqué par des flèches). **2.** Un *Toxoplasma* à l'intérieur d'un fibroblaste humain, préparant sa division. L'apex est riche en épitopes reconnus par le sérum anti-DGDG. Les deux cellules filles sont indiquées par des flèches et possèdent chacune un stock de galactolipides détecté en position apicale. **3.** Deux *Toxoplasma* à l'intérieur d'un fibroblaste humain, préparant leur division. **4.** Quatre *Toxoplasma* à l'intérieur d'un fibroblaste humain. **5.** Plus de huit *Toxoplasma* à l'intérieur d'un fibroblaste humain.

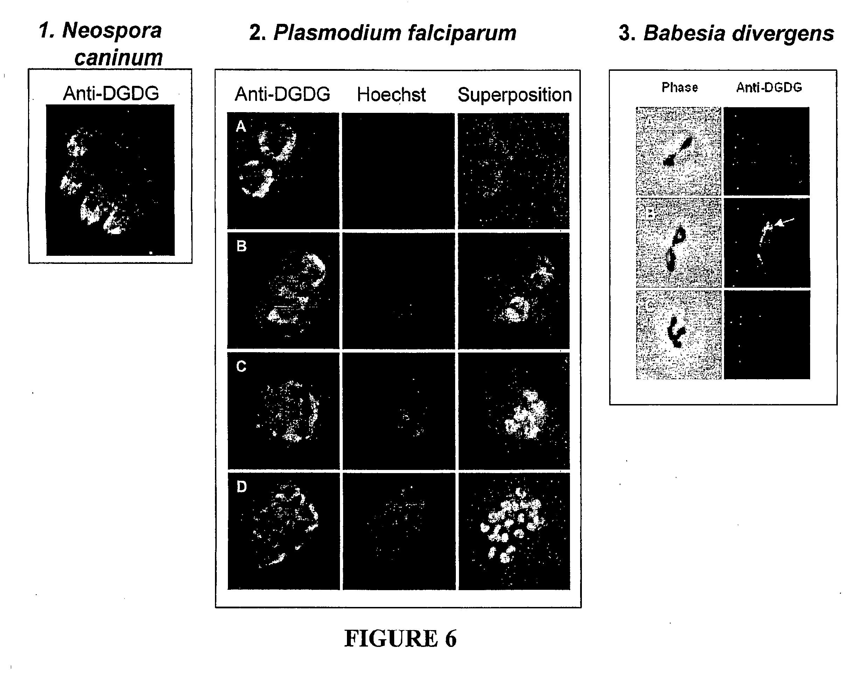

- la figure 6 illustre l'immunomarquage de cellules de *Néospora caninum* (1), *Plasmodium falciparum* (2) et *Babesia divergens* (3) à l'aide d'un sérum de lapin immunisé avec du DGDG purifié de chloroplastes d'épinard (Anti-DGDG). Les trois immunomarquages sont présentés pour des stades intracellulaires des parasites. Le marquage par la méthode de Hoechst présenté dans le cas de *Plasmodium* permet de repérer les noyaux des cellules parasitaires. Les cellules infectées par *Babesia divergens* sont visualisées, en parallèle, en microscopie de phase.

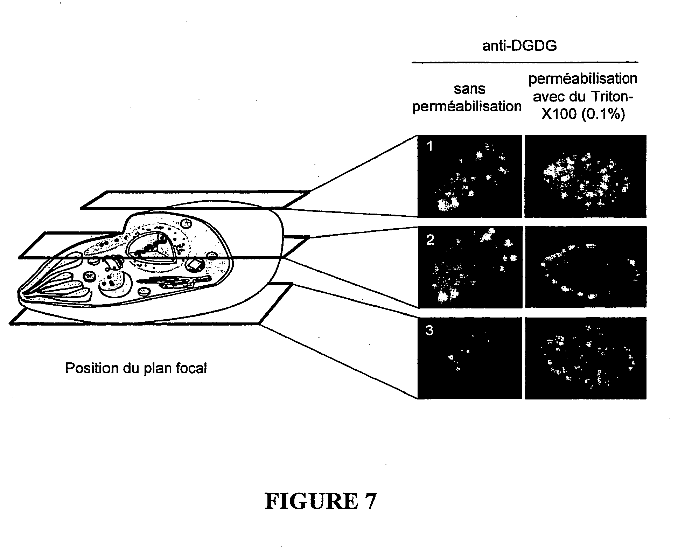

- la figure 7 illustre l'immunomarquage de la forme libre de *Toxoplasma gondii à* l'aide d'un sérum de lapin immunisé avec du DGDG purifié de chloroplastes d'épinard. Les immunomarquages ont été effectués à l'aide d'un sérum anti-DGDG dilué au 25^{ème} avec perméabilisation par le détergent Triton X-100 (images 1-3 à droite) ou sans perméabilisation (images 1-3 à gauche).

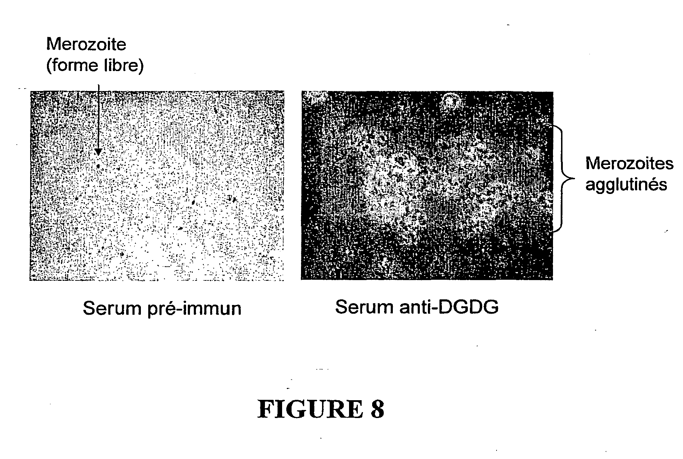

- la figure 8 illustre l'immunoagglutination de mérozoites (formes sanguines libres) de *Plasmodium falciparum* à l'aide d'un sérum de lapin immunisé avec du DGDG purifié de chloroplastes d'épinard (Sérum anti-DGDG). Le sérum préimmun ne montre aucune propriété agglutinante.

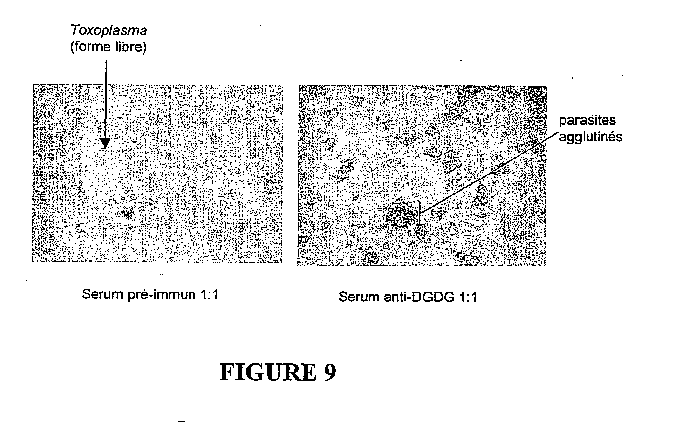

- la figure 9 illustre l'immunoagglutination de formes libres de *Toxoplasma gondii* à l'aide d'un sérum de lapin immunisé avec du DGDG purifié de chloroplastes d'épinard (Sérum anti-DGDG). Le sérum pré-immun ne montre aucune propriété agglutinante.

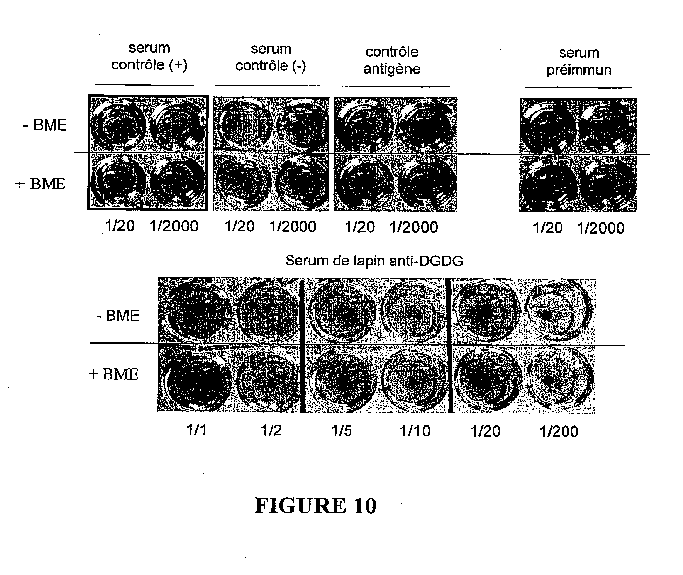

- la figure 10 illustre l'immunoagglutination de *Toxoplasma* à l'aide du kit Toxo-Screen DA^{®} de BIOMERIEUX. La partie supérieure montre le résultat de ce test pour des sérums contrôles, soit non-immunoagglutinant les toxoplasmes (Sérum contrôle (-) ; sédimentation des toxoplasmes en bouton ou en anneau), soit agglutinant des toxoplasmes sous forme de voile (sérum contrôle (-) ; puits encadrés). La partie inférieure montre un test réalisé en parallèle à l'aide de différentes dilutions de sérums de lapin (α-DGDG) ; le voile correspondant à une immunoagglutination est observé à partir de la dilution 1/2 du sérum anti-DGDG.

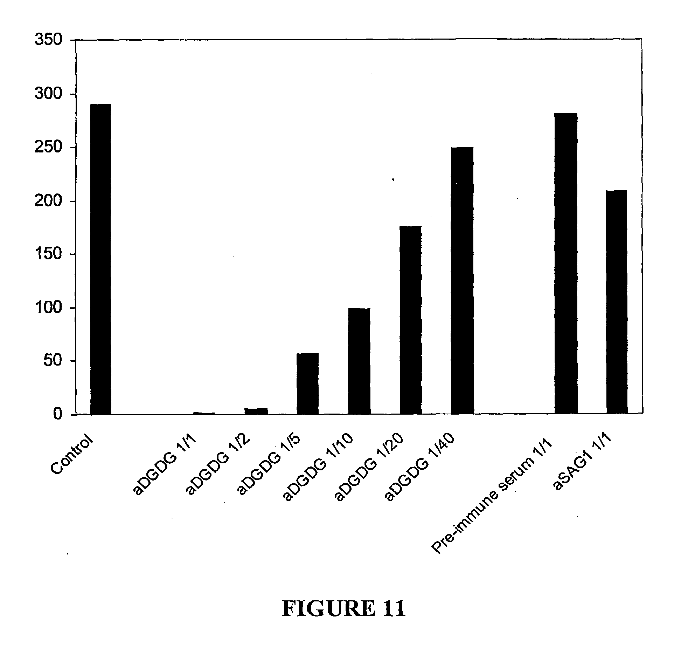

- la figure 11 illustre l'effet de l'anticorps anti-DGDG (aDGDG ; dilutions 1/40 à 1/1) sur l'invasion de cellules humaines par *Toxoplasma gondii*. Les cellules incubées avec *Toxoplasma gondii*, seul (Contrôle) ou en présence d'un sérum pré-immun ou d'anticorps dirigés contre l'antigène majeur de surface de *Toxoplasma gondii* SAG1 ou p30 (aSAG1), servent de contrôles.

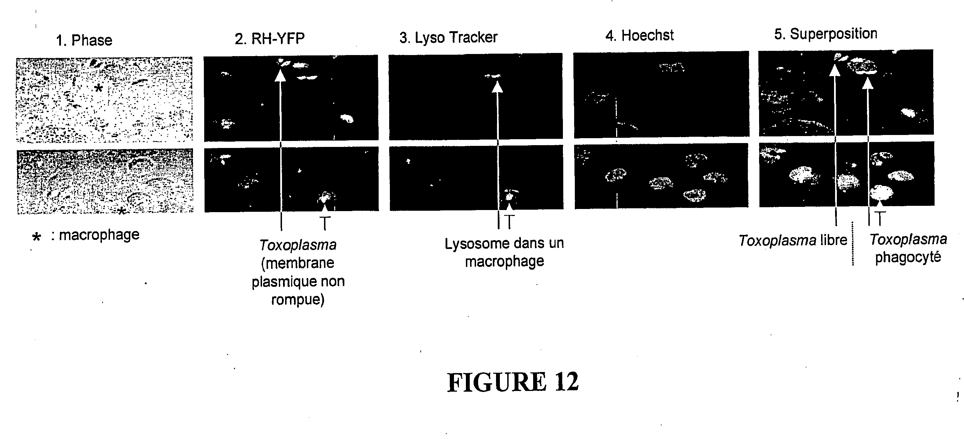

- la figure 12 illustre le principe du test d'opsonisation de cellules de *Toxoplasma gondii.* Des toxoplasmes exprimant la protéine fluorescente jaune YFP (lignée recombinante RH-YFP) sont incubés avec des macrophages de rats et la phagocytose des cellules de toxoplasmes par les macrophages est observée.
   1. microscopie de phase.
   2 à 5 microscopie de fluorescence : 2. visualisation de l'émission de fluorescence (540 nm) par les toxoplasmes recombinants (RH-YFP) après excitation à 488 nm. 3. marquage des lysosomes des macrophages générés par la phagocytose, à l'aide d'une sonde fluorescente rouge (Lyso-Tracker red, INVITROGEN).
   4. visualisation des noyaux des macrophages par la coloration d'Hoechst. 5. La superposition des fluorescences de la protéine YFP (fluorescence verte) et du Lyso-Tracker (fluorescence rouge) permet de détecter les parasites phagocytés par les macrophages (fluorescence jaune).

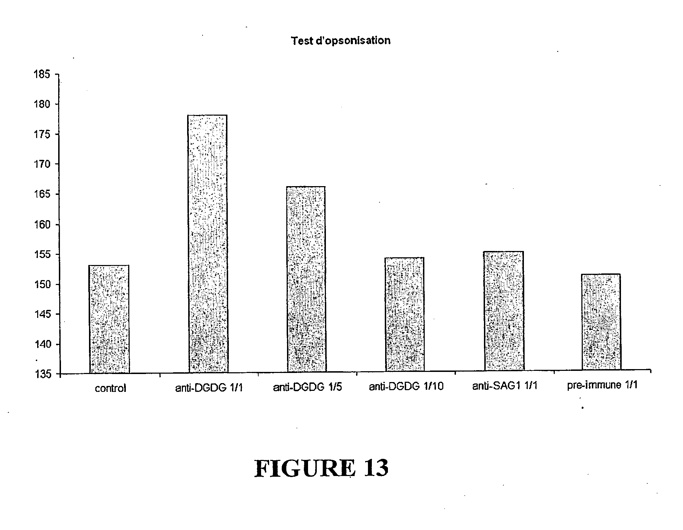

- la figure 13 illustre l'effet de l'anticorps anti-DGDG (aDGDG ; dilutions 1/10, 1/5 et 1/1) sur l'opsonisation des cellules de *Toxoplasma gondii.* Les macrophages incubés avec *Toxoplasma gondii,* seul (Contrôle) ou *Toxoplasma gondii* préalablement incubé avec un sérum pré-immun ou des anticorps dirigés contre l'antigène majeur de surface de *Toxoplasma gondii* SAG1 ou p30 (aSAG1), servent de contrôles.

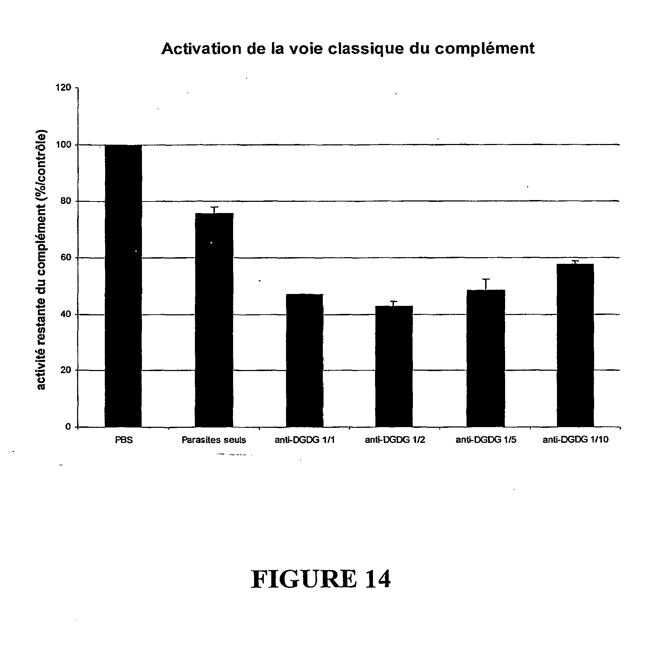

- la figure 14 illustre l'effet des anticorps anti-DGDG incubés en présence de *T*. *gondii* sur la voie classique d'activation du complément par mesure du temps nécessaire pour lyser 50% des érythrocytes sensibilisés (TH50). Les parasites (8.10⁶ par condition) ont été préincubés en présence de différentes dilutions d'un sérum de lapin anti-DGDG. Après élimination du milieu de prétraitement, les toxoplasmes sont mis en solution avec du sérum humain séronégatif pour la toxoplasmose (NHS, contenant les protéines du complément) puis avec des hématies sensibilisées. L'efficacité de chaque dilution de sérum à activer la voie classique du complément est appréciée par le temps nécessaire pour lyser 50% des hématies sensibilisées et par comparaison de ce temps avec le temps mesuré pour lyser 50% des hématies incubées en présence de PBS et de NHS. L'activité complément restante est exprimée par le ratio : TH50 contrôle (PBS+NHS+hématies) ./. TH50 de l'échantillon (parasites + sérum+ NHS + hématies) X 100. Les résultats correspondent aux mesures réalisées lors de trois expérimentations indépendantes en triplicats.

### Exemple 1 : Production d'anticorps dirigés contre le DGDG

### 1) Matériel et méthodes

### a) Extraction et purification du DGDG

Le DGDG est purifié à partir d'extraits de chloroplastes d'épinard, selon le protocole décrit dans Williams et al., J. Lipid Research, 1975, 16, 61-66 ou Jouhet et al., FEBS Letters, 2003, 544, 63-68.

### b) Immunisation des animaux avec le DGDG

Deux lapins et un rat ont été immunisés avec du DGDG purifié (2,5 mg par immunisation), selon le protocole suivant :
- une première immunisation (J₀) par voie sous-cutanée multisites (volume total de 1 ml) de 0,5 mg d'antigène dans 0,5 ml d'une solution de NaCl (0,9 %), émulsionné dans 0,5 ml d'adjuvant complet de Freund,
- une seconde immunisation (J₁₀) par voie sous-cutanée (1 ml) et intramusculaire (0,5 ml) de 0,5 mg d'antigène dans 0,75 ml d'une solution de NaCl (0,9 %), émulsionné dans 0,75 ml d'adjuvant de Freund incomplet,
- une troisième immunisation (J₂₁) par voie sous-cutanée (1 ml) et intramusculaire (0,5 ml) de 0,5 mg d'antigène dans 0,75 ml d'une solution de NaCl (0,9 %), émulsionné dans 0,75 ml d'adjuvant de Freund incomplet,
- une quatrième immunisation (J₃₆) par voie intramusculaire (0,5 ml) et intraveineuse (0,5 ml) : 0,17 mg d'antigène dans 0,25 ml d'une solution de NaCl (0,9 %), émulsionné dans 0,25 ml d'adjuvant de Freund incomplet, injecté par voie intramusculaire, et 0,33 mg d'antigène dans 0,5 ml d'une solution de NaCl (0,9 %), injecté par voie intraveineuse, et
- une cinquième immunisation (J₅₀) par voie intramusculaire (0,5 ml) et intraveineuse (0,5 ml) : 0,17 mg d'antigène dans 0,25 ml d'une solution de NaCl (0,9 %), émulsionné dans 0,25 ml d'adjuvant de Freund incomplet, injecté par voie intramusculaire et 0,33 mg d'antigène dans 0,5 ml d'une solution de NaCl (0,9 %), injecté par voie intraveineuse,

Les sérums ont été prélevés à J₅₇ ; les sérum prélevés avant l'immunisation (sérum pré-immuns) servent de contrôle.

### c) Immunoblot

Les lipides (extraits lipidiques ou lipides purifiés) dilués dans du chloroforme ou du butanol, sont déposés à la surface d'une membrane de nitrocellulose, incubés en présence des sérums dilués au 100^{ième}, et les complexes antigène-anticorps formés sont révélés à l'aide d'un anticorps secondaire couplé à la peroxidase et d'un substrat chimioluminescent (ECL, AMERSHAM), selon les protocoles classiques d'immunoblot.

### 2) Résultats

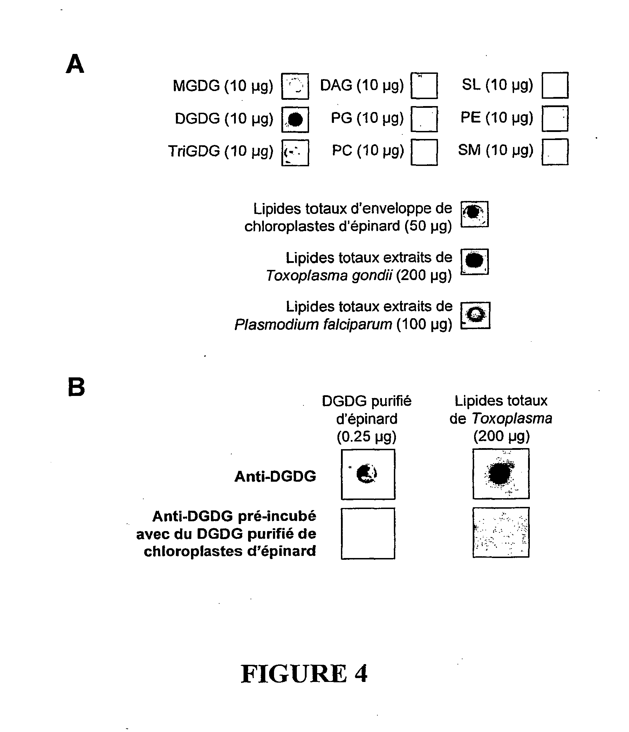

L'immunisation des animaux avec le digalactolipide de plante, DGDG, a permis d'obtenir des sérums actifs, contenant des anticorps reconnaissant le DGDG (Figure 4). L'immunisation d'un lapin avec l'autre galactolipide plastidial majeur des plantes, le MGDG, selon le même protocole, n'a pas permis d'obtenir de sérum actif.

### Exemple 2 : Analyse de la spécificité des anticorps anti-DGDG

### 1) Matériels et méthodes

L'activité des sérums dirigés contre le digalactolipide plastidial (DGDG) est analysée par immunoblot, selon le protocole décrit à l'exemple 1.

### 2) Résultats

Tous les sérums immuns ont une activité de liaison au DGDG (Figure 4A). Les sérums reconnaissent le DGDG de plantes (extraits d'épinards) et de parasites Apicomplexes (*Toxoplasma* et *Plasmodium* ; Figure 4A) alors que les sérums pré-immuns ne présentent aucune activité de liaison au DGDG.

L'activité de liaison des sérums immuns est spécifique du DGDG ; aucune liaison n'est détectée avec d'autres lipides (Figure 4A) et la liaison est abolie lorsque les sérums sont préalablement incubés en présence de DGDG (Figure 4B).

En outre, l'absence de réactivité des sérums anti-DGDG avec le MGDG et le tri-DGD indique que les anticorps dirigés contre le DGDG reconnaissent spécifiquement la partie digalactoside associée de façon covalente à la partie glycéro-lipide.

### Exemple 3 : Détection et localisation du DGDG dans les membranes cellulaires des parasites apicomplexes

### 1) Matériels et méthodes

Les parasites apicomplexes, sous forme libre ou intracellulaire sont immunomarqués par les sérums anti-DGDG de l'exemple 1 (directement ou après perméabilisation des membranes à l'aide d'une faible concentration de Triton-X100) et/ou marqués par le colorant de Hoechst qui colore les noyaux. Le marquage des parasites libres ou intracellulaires est analysé par microscopie de fluorescence.

### 2) Résultats

L'immunomarquage de parasites apicomplexes à l'aide des anticorps dirigés contre le digalactolipide plastidial (anticorps anti-DGDG de l'exemple 1) et la détection en microscopie de fluorescence, montrent que les cellules de parasites apicomplexes sont immunomarquées après perméabilisation des membranes à l'aide d'une faible concentration du détergent Triton-X100 (Figure 5). Le DGDG est toujours détecté dans les cellules de parasites et n'est jamais détecté sur la cellule humaine. Ces marquages montrent que le digalactolipide des parasites est localisé dans une structure qui se dédouble avant la division cellulaire (Figure 5, panneaux 2 et 3). Ce marquage est typique du complexe membranaire interne dont la fission précède la division cellulaire. Le marquage des parasites Apicomplexes est observé sur l'ensemble des stades parasitaires, chez *Toxoplasma* (Figure 5), *Plasmodium, Neospora* et *Babesia* (Figure 6).

L'immunomarquage des cellules parasitaires libres, avec ou sans perméation des membranes à l'aide du détergent Triton-X100 (Figure 7) montre dans les deux cas, un marquage en périphérie de la cellule, suivant le contour de la cellule lorsque le plan focal de l'observation est fixé de la partie la plus proche à la partie la plus lointaine de la cellule (voir schéma explicatif de la figure 7). Ces résultats indiquent que l'épitope reconnu par l'anticorps anti-DGDG est localisé, en plus du complexe membranaire interne, à la surface externe de la membrane plasmique des parasites apicomplexes.

### Exemple 4 : Immunoagglutination de parasites apicomplexes à l'aide de sérum anti-DGDG obtenu après immunisation à l'aide de digalactolipide de plantes

### 1) Matériels et méthodes

### a) Agglutination des parasites apicomplexes vivants

Des parasites vivants sont incubés avec différentes dilutions (1 :10 à 1 :1 dans du PBS) du sérum anti-DGDG ou du sérum pré-immun de l'exemple 1, préalablement inactivés 30 min à 37°C, et les parasites sont observés en microscopie optique.

### b) Agglutination directe d'antigènes apicomplexes sensibilisés

Le test d"immunoagglutination de *Toxoplasma gondii* est décrit dans Desmonts, G. et J.S. Remington, J. Clin. Microbiol., 1980, 11: 562-568. Ce test est le test de référence pour la détection des IgG anti-toxoplasmes (kit Toxo-Screen DA^{®}, référence 75 481, BIOMERIEUX).

L'antigène utilisé est une suspension de toxoplasmes formolés (souche Sabin), dilués dans un tampon albumine pH 8,95. Le kit comprend un sérum de chèvre positif, calibré par rapport à l'étalon OMS (CTR positif) et un sérum de chèvre négatif (CTR Négatif). L'agglutination est réalisée en plaques de microtitration à fond rond. Le test est réalisé en duplicat pour chaque sérum : absence d'agent réducteur (2-béta-Mercapto-Ethanol, 2-ME ou B-ME) sur la première ligne (- B-ME) ; présence de 2-ME sur la deuxième ligne (+ B-ME). L'agent réducteur permet de dénaturer les IgM et donc de rechercher leur présence dans le sérum.

Les sérums sont dilués dans du PBS et répartis dans les cupules, le 2-ME (25 µL; 0,2 mol/l) est ajouté dans les cupules de la deuxième ligne, puis la suspension d'antigène dilué au 1/5 (50 µL) est ajoutée dans toutes les cupules. Après homogénéisation, la plaque est recouverte d'une feuille autocollante et incubée 5 à 18 heures à température ambiante à l'abri des vibrations.
La lecture des plaques s'effectue de la façon suivante (Figure 10) :
- témoin antigène : sédimentation des toxoplasmes en bouton ou en anneau,
- réaction positive : agglutination des toxoplasmes sous forme de voile,
- réaction négative : sédimentation des toxoplasmes en bouton ou en anneau, et
- réaction limite : agglutination sous forme de voile tapissant la moitié du fond de la cupule.

### 2) Résultats

Les propriétés agglutinantes des sérums obtenus après immunisation de lapins avec du DGDG purifié de plantes ont été testées sur des parasites apicomplexes vivants ou formolés.
La figure 8 montre l'agglutination de mérozoites (forme libre) vivants de l'agent du paludisme (*Plasmodium falciparum*)*,* avec un sérum anti-DGDG dilué au 5^{ième}. La figure 9 montre l'agglutination de formes libres de *Toxoplasma gondii* avec un sérum anti-DGDG ; l'immunoagglutination est visible dès la concentration 1 :10 et maximale à la dilution 1 :1. Le sérum préimmun ne montre aucune propriété agglutinante.
   La figure 10 montre l'agglutination de toxoplasmes formolés, avec un sérum anti-DGDG dilué au demi et au 20^{ième}. Ce sérum contient des IgM car le B-ME diminue l'agglutination de 50 %.

### Exemple 5 : Les anticorps anti-DGDG inhibent l'infection des cellules humaines par Toxoplasma gondii.

### 1) Matériels et méthodes

L'infection de cellules humaines par *Toxoplasma gondii* est analysée par un test de dosage de la béta-galactosidase dans des cellules de la lignée HFF (Human Foreskin Fibroblasts) infectées par une souche de toxoplasme recombinant comprenant le gène LacZ sous le contrôle du promoteur SAG1, selon la méthode décrite par McFadden, D.C. et al., Antimicrobial agents and chemotherapy, 1997, 41, 1849-1853 ; Seeber, F. and Boothroyd, J.C., Gene, 1996, 169, 39-45.

L'effet des anticorps anti-DGDG sur l'infection des cellules HHF par *Toxoplasma gondii* est testé à des dilutions de 1 : 40 à 1 :1. Les cellules incubées avec *Toxoplasma gondii*, seul (Contrôle) ou en présence d'un sérum pré-immun ou bien d'anticorps dirigés contre l'antigène majeur de surface de *Toxoplasma gondii* SAG1 ou p30 (aSAG1) servent de contrôles.

### 2) Résultats

La figure 11 montre que les anticorps anti-DGDG inhibent l'infection des cellules humaines par *Toxoplasma gondii* ; l'effet est visible dès la concentration 1 :20 et maximal à la dilution 1 :2. Le sérum préimmun est sans effet et les anticorps anti-SAG1 ont un effet très faible.

### Exemple 6 : Les anticorps anti-DGDG augmentent l'opsonisation de Toxoplasma gondii.

### 1) Matériels et méthodes

Des toxoplasmes recombinants RH-YFP (Gubbels et al., Antimicrobial agents and Chemotherapy, 47, 309-306) sont incubés avec différents anticorps (anti-DGDG 1 :1, 1 :5 et 1 :10 ; sérum pré-immun (1 :1) et anticorps anti-SAG1 (1 :1)), pendant 30 min à 37°C, sous agitation (roue orbitale), centrifugés 10 min à 2000 rpm (lavage), puis comptés.

Des macrophages de rat sont déposés sur des lamelles (5000 cellules/lamelle) recouvertes de polylysine (1 mg/ml) et incubés 2 h avec du Lysotracker Red DND-99 (dilution 1/20000 ; IN VITROGEN). Les macrophages sont ensuite infectés avec les toxoplasmes (50 toxoplasma/macrophage), pendant 2 h à 4 h à 37 °C (phagocytose/ invasion) en présence de Lysotracker, lavés puis fixés 20 min avec 5 % PFA et colorés au Hoechst (1/15000).

Les fluorescences de la protéine YFP et du lysotracker sont analysés en microscopie confocale.

### 2) Résultats

Le test d'opsonisation *in vitro* montre une stimulation de 20 % en présence d'anticorps anti-DGDG (figure 13).

### Exemple 7 : Les anticorps anti-DGDG activent la voie classique du complément grâce à l'opsonisation du parasite

La capacité du sérum anti-DGDG (exemple 1) à activer la voie classique du complément a été évaluée. Le système du complément est une cascade biochimique du système immunitaire inné impliquant 9 protéines principales (C1 à C9). L'activation de ce système permet la destruction de pathogènes et le recrutement d'un grand nombre d'acteurs du système immunitaire pour une réponse régulée (pour revue générale, Janeway et al., Annu Rev Immunol., 2002, 20, 197-216). La voie classique est activée par le complexe antigène-anticorps. L'interaction du complexe protéique C1 avec le fragment constant de deux (ou plusieurs) IgG (Ig1, Ig2 et Ig3) ou encore d'une IgM pentamérique permet d'initier la cascade. Le complexe antigène-anticorps-C1 permet de cliver les protéines C4 et C2, qui s'associent puis clivent la protéine C3 en C3a et C3b. C3b se fixe sur la membrane du pathogène entraînant la formation, avec d'autres protéines du complément, d'un pore aboutissant à la lyse de la cellule touchée. C3b joue aussi un rôle en tant qu'opsonine et facilite la phagocytose par les macrophages.

### 1) Matériel et méthodes

L'activation de la voie classique du complément par les anticorps anti-DGDG de l'exemple 1 a été mesurée selon le protocole de détermination dite de la « TH50 » (temps nécessaire pour observer 50% d'hémolyse, Abbal et al., Complement Inflammation, 1991, 8, 92-103) avec détermination de la présence de la protéine C4 hémolytique (Gaither et al., J. Immunol., 1974, 113, 574-583). Brièvement, des érythrocytes de mouton sont sensibilisés avec un anticorps anti-érythrocytaire pour former une particule sensibilisée. Une solution de sérum humain (NHS) est ensuite ajoutée à la solution contenant cette particule sensibilisée. Le NHS contient l'ensemble des protéines du complément, y compris le complexe C1q nécessaire à l'initiation de la voie classique du complément. Au contact de l'érythrocyte sensibilisé, C1q initie la cascade du complément, ce qui entraîne la lyse des hématies. Cette lyse et plus particulièrement, la libération de l'hémoglobine qui en résulte, est mesurée au cours du temps par spectrométrie et le point d'inflexion de la courbe constitue le temps nécessaire à la lyse de 50% des hématies (TH50). Cette mesure constitue le TH50 de référence.

Des parasites extracellulaires fraîchement lysés (8.10⁶ par condition) ont été incubés en présence de plusieurs dilutions du sérum de lapin anti-DGDG (1/1, 1/2, 1/5, 1/10, 1/20, 1/50 et 1/100) ainsi qu'avec du sérum pré-immun. Les parasites ont ensuite été rincés en PBS puis incubés 1 h à 37°C avec du NHS séronégatif à la toxoplasmose. Lors de cette étape, les protéines du complément peuvent en partie être consumées par le complexe sérum-antigène. La solution NHS-serum-antigène est ensuite mélangée à la solution contenant les hématies sensibilisées. La mesure du TH50 est alors effectuée. La capacité d'un sérum à activer le complément conduit à une augmentation du TH50 de référence, liée à la baisse du nombre d'érythrocytes lysés. Elle peut donc être quantifiée par rapport au contrôle. Le TH50 de référence a été mesuré dans un mélange hématies sensibilisées-NHS-PBS. De plus, du fait que certains pathogènes sont capables d'activer le complément (voie alternative et voie des lectines) par leur seule présence, une mesure de contrôle du TH50 a été réalisée sur des parasites extracellulaires sans prétraitement au sérum avec le NHS.

### 2) Résultats

Les mesures du TH50 (Figure 14) montrent que le prétraitement des toxoplasmes par le sérum de lapin anti-DGDG (dilutions 1/1 à 1/10) active la voie classique du complément de manière significative. Le maximum de cette activation est atteint à la dilution 1/2 (42% d'activité complément restante seulement, par rapport au 100% de la référence parasites en PBS). C'est à cette dilution que les conditions de stoechiométrie semblent être les plus favorables à la réaction. Le prétraitement par le sérum anti-DGDG dilué au 1/10, au 1/5 ou au 1/1 conduit à une diminution marquée de l'activité complément restante mais proportionnelle à la dilution, par rapport au contrôle PBS. Il est intéressant de noter que les parasites seuls, incubés en présence de NHS et d'hématies sensibilisées induisent une diminution de cette activité complément restante, par rapport au contrôle PBS (77% d'activité seulement). Ces résultats suggèrent que le toxoplasme pourrait activer la cascade du complément indépendamment de la présence d'anticorps spécifiques : il pourrait donc activer la voie alterne ou la voie des lectines.

## Revendications

1. Utilisation d'un digalactolipide purifié comprenant le digalactose 6-O-(α-D-galactopyrannosyl)-β-D-galactopyrannose associé de façon covalente à un lipide comme antigène pour la préparation d'un vaccin destiné à la prévention ou au traitement des infections par les parasites apicomplexes chez l'homme ou l'animal.

2. Utilisation selon la revendication 1, **caractérisée en ce que** ledit digalactolipide est le 1,2-diacyl-(α-D-galactopyranosyl-(1'→6')-β-D-galactopyranosyl-(1'→ 3)-sn-glycerol.

3. Utilisation d'un anticorps dirigé contre le digalactolipide purifié tel que défini à la revendication 1 ou 2 ou d'un fragment fonctionnel dudit anticorps comprenant au moins les domaines variables des chaînes lourdes et légères, pour la préparation d'un médicament destiné à la prévention ou au traitement des infections par les parasites apicomplexes chez l'homme ou l'animal.

4. Utilisation selon la revendication 3, **caractérisée en ce que** ledit anticorps ou fragment d'anticorps est choisi parmi les anticorps monoclonaux, les anticorps polyclonaux, les anticorps chimériques et les fragments Fab, Fv et scFv des anticorps précédents.

5. Utilisation selon l'une quelconque des revendications 1 à 4, **caractérisée en ce que** lesdits parasites sont des pathogènes sélectionnés dans le groupe constitué par *Plasmodium, Babesia, Toxoplasma, Neospora, Cryptosporidium, Theileria, Sarcocystis, Eimeria* et *Gregarina.*

6. Méthode *in vitro* de détection d'une infection par un parasite apicomplexe, comprenant l'immunodétection des parasites apicomplexes ou des anticorps spécifiques desdits parasites présents dans un échantillon biologique issu d'un individu, selon les étapes consistant en :
(a) la mise en contact dudit échantillon biologique avec au moins un antigène, un anticorps ou un fragment fonctionnel d'anticorps comprenant au moins les domaines variables des chaînes lourdes et légères tels que définis à l'une quelconque des revendications 1 à 4, soit directement, soit en même temps que ou après une étape de perméabilisation membranaire dudit échantillon biologique, et
(b) la révélation des complexes antigène-anticorps formés en (a).

7. Méthode selon la revendication 6, **caractérisée en ce que** ledit antigène, anticorps ou fragment d'anticorps de l'étape a) est couplé à un marqueur.

8. Méthode selon la revendication 6 ou 7, **caractérisée en ce que** ledit antigène, anticorps ou fragment d'anticorps de l'étape a) est immobilisé sur un support.

9. Méthode selon l'une quelconque des revendications 6 à 8, **caractérisée en ce que** l'étape (a) comprend la mise en contact dudit échantillon biologique, successivement avec au moins un premier anticorps de capture dirigé contre l'antigène digalactolipidique ou un fragment fonctionnel dudit anticorps tels que définis à la revendication 3 ou 4 et au moins un second anticorps spécifique du parasite à détecter et différent du premier anticorps ou un fragment fonctionnel dudit second anticorps comprenant au moins les domaines variables des chaînes lourdes et légères.

10. Méthode selon la revendication 9, **caractérisée en ce que** le second anticorps est un anticorps spécifique du genre ou de l'espèce du parasite à détecter.

11. Méthode selon l'une quelconque des revendications 6 à 10, **caractérisée en ce que** ledit parasite est un pathogène sélectionné dans le groupe constitué par *Plasmodium, Babesia, Toxoplasma, Neospora, Cryptosporidium, Theileria, Sarcocystis, Eimeria* et *Gregarina.*

12. Digalactolipide purifié tel que défini à la revendication 1 ou 2 pour utilisation comme médicament destiné à la prévention ou au traitement des infections par les parasites apicomplexes.

13. Anticorps ou fragment d'anticorps tels que définis à la revendication 3 ou 4 pour utilisation comme médicament destiné à la prévention ou au traitement des infections par les parasites apicomplexes.

## Claims

1. Use of a purified digalactolipid comprising the digalactose 6-O-(α-D-galactopyranosyl)-β-D-galactopyranose covalently associated with a lipid, as an antigen for the preparation of a vaccine intended for preventing or treating infections with apicomplexan parasites in humans or animals.

2. Use according to Claim 1, **characterized in that** said digalactolipid is 1,2-diacyl-(α-D-galactopyranosyl)-(1'→6')-β-D-galactopyranosyl-(1'→3)-sn-glycerol.

3. Use of an antibody directed against the purified digalactolipid as defined in Claim 1 or 2 or of a functional fragment of said antibody comprising at least the variable domains of the heavy and light chains, for the preparation of a medicament intended for preventing or treating infections with apicomplexan parasites in humans or animals.

4. Use according to Claim 3, **characterized in that** said antibody or antibody fragment is chosen from monoclonal antibodies, polyclonal antibodies, chimeric antibodies and the Fab, Fv and scFv fragments of the above antibodies.

5. Use according to any one of Claims 1 to 4, **characterized in that** said parasites are pathogens selected from the group constituted of *Plasmodium, Babesia, Toxoplasma, Neospora, Cryptosporidium, Theileria, Sarcocystis, Eimeria* and *Gregarina.*

6. *In vitro* method for detecting an infection with an apicomplexan parasite, comprising the immunodetection of the apicomplexan parasites, or of the antibodies specific for said parasites, that are present in a biological sample taken from an individual, according to the steps consisting in:
(a) bringing said biological sample into contact with at least one antigen, one antibody or one functional antibody fragment comprising at least the variable domains of the heavy and light chains, as defined in any one of Claims 1 to 4, either directly, or at the same time as or after a step of membrane permeabilization of said biological sample, and
(b) revealing the antigen-antibody complexes formed in (a).

7. Method according to Claim 6, **characterized in that** said antigen, antibody or antibody fragment of step a) is coupled to a label.

8. Method according to Claim 6 or 7, **characterized in that** said antigen, antibody or antibody fragment of step a) is immobilized on a support.

9. Method according to any one of Claims 6 to 8, **characterized in that** step (a) comprises bringing said biological sample into contact successively with at least one first capture antibody directed against the digalactolipid antigen or a functional fragment of said antibody, as defined in Claim 3 or 4, and at least one second antibody specific for the parasite to be detected and different from the first antibody or a functional fragment of said second antibody comprising at least the variable domains of the heavy and light chains.

10. Method according to Claim 9, **characterized in that** the second antibody is an antibody specific for the genus or for the species of the parasite to be detected.

11. Method according to any one of Claims 6 to 10, **characterized in that** said parasite is a pathogen selected from the group constituted of *Plasmodium, Babesia, Toxoplasma, Neospora, Cryptosporidium, Theileria, Sarcocystis, Eimeria* and *Gregarina.*

12. Purified digalactolipid as defined in Claim 1 or 2, for use as a medicament intended for preventing or treating infections with apicomplexan parasites.

13. Antibody or antibody fragment as defined in Claim 3 or 4, for use as a medicament intended for preventing or treating infections with apicomplexan parasites.

## Patentansprüche

1. Verwendung eine gereinigten Digalactolipids, umfassend die Digalactose 6-O-(a-D-Galactopyrannosyl)-β-D-galactopyrannose, die kovalent mit einem Lipid assoziiert ist, als Antigen, zur Herstellung eines Impfstoffs zur Vorbeugung oder Behandlung von Infektionen mit apikomplexen Parasiten bei Menschen oder Tieren.

2. Verwendung nach Anspruch 1, **dadurch gekennzeichnet, dass** das Digalactolipid 1,2-Diacyl-(α-D-galactopyranosyl-(1'→6')-β-D-galactopyranosyl)-(1'→3)-sn-glycerin ist.

3. Verwendung eines Antikörpers gegen das gereinigte Galactolipid nach Anspruch 1 oder 2 oder eines funktionellen Fragments des Antikörpers, umfassend wenigstens die variablen Domänen der schweren oder leichten Ketten, zur Herstellung eines Medikaments zur Vorbeugung oder Behandlung von Infektionen mit apikomplexen Parasiten bei Menschen oder Tieren.

4. Verwendung nach Anspruch 3, **dadurch gekennzeichnet, dass** der Antikörper oder das Antikörperfragment ausgewählt sind aus monoklonalen Antikörpern, polyklonalen Antikörpern, chimären Antikörpern und den Fragmenten Fab, Fv und scFv der vorgenannten Antikörper.

5. Verwendung nach einem der Ansprüche 1 bis 4, **dadurch gekennzeichnet, dass** die Parasiten Pathogene sind, die ausgewählt sind aus der Gruppe bestehend aus *Plasmodium, Babesia, Toxoplasma, Neospora, Cryptosporidium, Theileria, Sarcocystis, Eimeria* und *Gregarina.*

6. in-vitro-Verfahren zum Nachweisen einer Infektion mit einem apikomplexen Parasiten, umfassend einen Immunnachweis von apikomplexen Parasiten oder von für diese Parasiten spezifischen Antikörpern in einer biologischen Probe eines Subjekts, wobei die Schritte umfassen:
a) In-Kontakt-bringen der biologischen Probe mit wenigstens einem Antigen, einem Antikörper oder einem funktionellen Antikörperfragment, umfassend wenigstens die variablen Domänen der schweren und leichten Ketten nach einem der Ansprüche 1 bis 4, entweder direkt, oder gleichzeitig oder nach einem Schritt der Membranpermeabilisation der biologischen Probe, und
b) Anzeigen der in a) gebildeten Antigen-Antikörper-Fragmente.

7. Verfahren nach Anspruch 6, **dadurch gekennzeichnet, dass** das Antigen, der Antikörper oder das Antikörperfragment aus Schritt a) an einen Marker gekoppelt sind.

8. Verfahren nach Anspruch 6 oder 7, **dadurch gekennzeichnet, dass** das Antigen, der Antikörper oder das Antikörperfragment aus Schritt a) auf einem Träger immobilisiert sind.

9. Verfahren nach einem der Ansprüche 6 bis 8, **dadurch gekennzeichnet, dass** der Schritt a) Probe aufeinanderfolgend In-Kontakt-bringen der biologischen Probe mit wenigstens einem ersten Fängerantikörper gegen das digalactolipidische Antigen oder einem funktionellen Antikörperfragment nach Anspruch 3 oder 4 und wenigstens einem zweiten Antikörper, der für den nachzuweisenden Parasiten spezifisch ist und sich von dem ersten Antikörper unterscheidet, oder einem funktionellen Fragment des zweiten Antikörpers, umfassend wenigstens die variablen Domänen der schweren und leichten Ketten, umfasst.

10. Verfahren nach Anspruch 9, **dadurch gekennzeichnet, dass** der zweite Antikörper ein Antikörper ist, der spezifisch für die Gattung oder Art des nachzuweisenden Parasiten ist.

11. Verfahren nach einem der Ansprüche 6 bis 10, **dadurch gekennzeichnet, dass** der Parasit ein Pathogen ist, das ausgewählt ist aus der Gruppe bestehend aus *Plasmodium, Babesia, Toxoplasma, Neospora, Cryptosporidium, Theileria, Sarcocystis, Eimeria* und *Gregarina.*

12. Gereinigtes Digalactolipid nach einem der Ansprüche 1 oder 2 zur Verwendung als Medikament zur Vorbeugung oder Behandlung von Infektionen mit apikomplexen Parasiten.

13. Antikörper oder Antikörperfragment nach einem der Ansprüche 3 oder 4 zur Verwendung als Medikament zur Vorbeugung oder Behandlung von Infektionen mit apikomplexen Parasiten.
